# EUROPÄISCHE PATENTSCHRIFT

(11) **EP 0 520 210 B1**
(45) Veröffentlichungstag und Bekanntmachung des Hinweises auf die Patenterteilung: **23.04.1997**
(21) Anmeldenummer: 92109267.2
(22) Anmeldetag: 02.06.1992
(51) Int. Cl.: C07D 229/00, C07D 251/34, C08G 18/79, C08G 18/02

(54) **Verfahren zur Herstellung von oligomeren Polyisocyanaten und ihre Verwendung**
Process for the preparation of oligomer polyisocyanates and their use
Procédé pour la préparation de polyisocyanates oligomères et leur utilisation

(30) Priorität: 15.06.1991 DE 4119753
(43) Veröffentlichungstag der Anmeldung: 30.12.1992
(73) Patentinhaber: BAYER AG, 51368 Leverkusen (DE)
(72) Erfinder: Scholl, Hans Joachim, Dr., W-5000 Köln 80 (DE)

(56) Entgegenhaltungen:
- EP-A- 0 337 116
- EP-A- 0 377 177
- DE-A- 1 670 270
- DE-A- 3 432 081

## Beschreibung

Die Erfindung betrifft ein neues Verfahren zur Herstellung von oligomeren Polyisocyanaten durch Oligomerisierung eines Teils der Isocyanatgruppen von organischen Polyisocyanaten unter Abbruch der Reaktion mit bestimmten silylierten Säuren und die Verwendung der Verfahrensprodukte, gegebenenfalls in mit Blockierungsmitteln für Isocyanatgruppen blockierter Form, als Polyisocyanatkomponente bei der Herstellung von Polyurethankunststoffen.

Unter "oligomeren Polyisocyanaten" sind im Rahmen der Erfindung Uretdiongruppen und daneben gegebenenfalls zusätzlich Isocyanurat- und/oder Urethangruppen aufweisende Polyisocyanate zu verstehen, wie sie bei der katalytischen Dimerisierung von organischen Polyisocyanaten, insbesondere Diisocyanaten, gegebenenfalls unter Mitverwendung von alkoholischen Cokatalysatoren entstehen. Die Urethangruppen entstehen dabei durch Reaktion eines Teils der Isocyanatgruppen mit den alkoholischen Cokatalysatoren. Insbesondere bei der bevorzugten Verwendung von tert. Phosphinen als Katalysatoren ist die Frage, ob bei der Umsetzung vorwiegend Uretdiongruppen aufweisende Dimerisierungsprodukte oder vorwiegend Isocyanuratgruppen aufweisende Trimerisierungsprodukte entstehen im wesentlichen vom Grad der Umsetzung und der Temperaturführung abhängig (vgl. A. Farkas und G.A. Mills, Adv. Catal. 13 (1962), Seiten 393 ff.).

Für eine reproduzierbare großtechnische Produktion ist es jedoch unerläßlich, die Dimerisierungs- und/oder Trimerisierungsreaktion, d.h. die "Oligomerisierungsreaktion", genau und schnell an einem vorbestimmten Punkt zu beenden.

Gemäß DE-OS 3 432 081 erfolgt die Abstufung der Reaktion durch Desaktivierung der eingesetzten Katalysatoren mittels Sulfonylisocyanaten, insbesondere Tosylisocyanat. Tosylisocyanat ist als Katalysatorgift zwar den seinerzeit vorbekannten Katalyatorengiften, wie sie in der DE-OS 3 432 081 besprochen werden, überlegen, jedoch sind die zwischen Tosylisocyanat als Katalysatorgift und Katalysator gebildeten Addukte offensichtlich immer noch so labil, daß der Katalysator rückgespalten werden kann und daher überäquimolare Mengen an Sulfonylisocyanat empfohlen werden. In der Tat ist, wie nachstehender Vergleichsversuch zeigt, der Zusatz von Tosylisocyanat in äquivalenten Mengen, bezogen auf die Menge des eingesetzten Katalysators, unzureichend, um eine zuverlässige Abstoppung der Reaktion zu gewährleisten.

Es war daher die der Erfindung zugrunde liegenden Aufgabe, ein neues Verfahren zur Oligomerisierung (= Dimerisierung und/oder Trimerisierung) von organischen Polyisocyanaten, insbesondere Diisocyanaten unter Verwendung von bekannten Katalysatoren des Standes der Technik zur Verfügung zu stellen, welches eine einwandfreie Abstoppung der Reaktion beim jeweils erwünschten Umsetzungsgrad gestattet.

Diese Aufgabe konnte mit dem nachstehend näher beschriebenen erfindungsgemäßen Verfahren gelöst werden.

Gegenstand der Erfindung ist ein Verfahren zur Herstellung von oligomeren Polyisocyanaten durch Oligomerisierung eines Teils der Isocyanatgruppen von organischen Polyisocyanaten in Gegenwart von die Dimerisierung von Isocyanatgruppen beschleunigenden Katalysatoren und Abbruch der Oligomerisierungsreaktion bei einem Umsatz von 5 bis 45 % der im Ausgangsgemisch vorliegenden Isocyanatgruppen durch Zugabe eines Katalysatorengifts, dadurch gekennzeichnet, daß man als Katalysatorengift eine silysierte Säure der Formel

X-[Si(CH₃)₃]ₙ

verwendet, wobei
- X: für den neutralen Säurerest steht, wie er durch Entfernung der aciden Wasserstoffatome aus einer n-basischen Säure mit einem pKa-Wert von maximal 3 erhalten wird, wobei Halogenwasserstoffsäuren ausgenommen sind, und
- n: für eine ganze Zahl von 1 bis 3 steht.

Gegenstand der Erfindung ist auch die Verwendung der nach diesem Verfahren erhaltenen, oligomeren Polyisocyanate, gegebenenfalls im Gemisch mit nicht umgesetztem Ausgangspolyisocyanat und/oder gegebenenfalls in mit Blockierungsmitteln für Isocyanatgruppen blockierter Form als Polyisocyanatkomponente bei der Herstellung von Polyurethankunststoffen nach dem Isocyanat-Polyadditionsverfahren.

Als Ausgangsmaterialien für das erfindungsgemäße Verfahren können beliebige organische Polyisocyanate, insbesondere Diisocyanate eingesetzt werden. Beispielhaft genannt seien insbesondere aliphatische Diisocyanate wie 1,6-Diisocyanatohexan (HDI), 1-Isocyanato-3,3,5-trimethyl-5-isocyanatomethyl-cyclohexan (IPDI), 4,4'-Diisocyanato-dicyclohexylmethan (HMDI) und beliebige Gemische derartiger aliphatischer Diisocyanate. Auch aromatische Diisocyanate wie 2,4- und/oder 2,6-Diisocyanatotoluol (TDI), 2,2'-, 2,4'- und/oder 4,4'-Diisocyanato-diphenylmethan (MDI) bzw. beliebige Gemische derartiger aromatischer Diisocyanate können erfindungsgemäß als Ausgangsmaterial eingesetzt werden.

Als Dimerisierungskatalysatoren können beliebige Dimerisierungskatalysatoren des Standes der Technik eingesetzt werden. Beispielsweise in Betracht kommen die Dimerisierungskatalysatoren gemäß DE-OS 3 030 513, DE-OS 3 432 081, DE-OS 1 670 720, DE-OS 3 420 114 oder DE-OS 3 739 549. Vorzugsweise werden jedoch beim erfindungsgemäßen Verfahren tertiäre Phosphine, insbesondere aliphatische, araliphatische oder gemischt aliphatische-aromatische Phosphine des Molekulargewichtsbereichs 76 bis 500 eingesetzt. Beispiele geeigneter tert.

Phosphine sind Triethylphosphin, Dibutylethylphosphin, Tri-n-propylphosphin, Tri-iso-propylphosphin, Tri-tert-butylphosphin, Tribenzylphosphin, Benzyldimethylphosphin, Dimethylphenylphosphin, Tri-n-butylphosphin, Tri-iso-butylphosphin, Triamylphosphin oder Trioctylphosphin. Tri-(n-butyl)-phosphin ist besonders gut als Katalysator für das erfindungsgemäße Verfahren geeignet. Die Menge des eingesetzten Katalysators hängt von der Reinheit des organischen Ausgangsisocyanats ab. Die jeweils notwendige Katalysatormenge läßt sich daher am einfachsten in einem Vorversuch bestimmen.

Bei aliphatisch gebundenen NCO-Gruppen beträgt die Menge normalerweise 0,1 bis 1 Gew.-% Katalysator, bei aromatisch gebundenen NCO-Gruppen 0,01 bis 0,1 Gew.-% bezogen auf Ausgangsisocyanat.

Oftmals ist es zweckmäßig, die katalytische Wirkung durch eine geringe Menge an cokatalytisch wirkenden Urethangruppen zu unterstützen. Eine derartige Cokatalyse ist beispielsweise durch Zusatz einer geringen Menge eines Alkohols (beispielsweise 0,01 bis 1 Gew.-%, bezogen auf das Gewicht des eingesetzten Ausgangsisocyanats) erreichbar, da die zugesetzten Alkohole sofort mit dem im Überschuß vorliegenden Ausgangsisocyanat unter Urethanbildung abreagieren. Geeignete derartige potentielle Cokatalysatoren sind beispielsweise Methanol, Ethanol, 2-Ethylhexanol-1 oder 2-Ethylhexandiol-1,3. Die Alkohole können gleichzeitig mit dem Katalysator oder vorab zugesetzt werden.

Die vorzugsweise unter Inertgasatmosphäre ablaufende Oligomerisierungsreaktion wird vorzugsweise lösungsmittelfrei innerhalb des Temperaturbereichs von 0 bis 100°C, insbesondere von 20 bis 80°C durchgeführt, kann jedoch auch in Gegenwart von inerten Lösungsmitteln wie z.B. Kohlenwasserstoffen wie Toluol, Chlorbenzol oder Xylol oder Estern, wie z.B. Butylacetat, erfolgen.

Die Reaktion wird im allgemeinen bei Erreichen eines Oligomerisierungsgrades von 5 bis 45, vorzugsweise 10 bis 40 %, abgebrochen. Dies entspricht Produktausbeuten von etwa 10 bis 90, vorzugsweise 20 bis 80 Gew.-%. Unter "Oligomerisierungsgrad" ist hierbei der Prozentsatz der Isocyanatgruppen zu verstehen, die während der Umsetzung, d.h. insbesondere der Dimerisierung, sowie gegebenenfalls Trimerisierung, sowie gegebenenfalls in untergeordnetem Umfang unter Urethanisierung abreagieren. Der Oligomerisierungsgrad kann während der Reaktion beispielsweise durch fortlaufende Bestimmung des Brechungsindex' oder des NCO-Gehalts des Reaktionsansatzes verfolgt werden.

Im allgemeinen werden die Reaktionsbedingungen und der Zeitpunkt des Abbruchs der Reaktion bei der Durchführung des erfindungsgemäßen Verfahrens so gewählt, das als Reaktionsprodukte vor allem Dimerisierungsprodukte (Uretdione) der als Ausgangsmaterial eingesetzten Ausgangspolyisocyanate anfallen, die allenfalls untergeordnete Mengen an Trimerisierungsprodukten (Isocyanuraten) enthalten. Da diese im allgemeinen in molarem Unterschuß vorliegen, - das Molverhältnis von Uretdiongruppen zu Isocyanuratgruppen in den erfindungsgemäßen Verfahrensprodukten liegt vorzugsweise bei mindestens 2:1 - wäre es auch gerechtfertig, den Reaktionsablauf als "Dimerisierung" zu bezeichnen.

Der erfindungswesentliche Punkt besteht nun darin, den genannten Abbruch der Reaktion durch Zusatz der erfindungswesentlichen Katalysatorengifte zu bewirken. Bei diesen erfindungswesentlichen Katalysatorengifte handelt es sich um silylierte Säuren der Formel

X-[Si(CH₃)₃]ₙ.

In dieser Formel haben X und n die bereits obengenannte Bedeutung. Vorzugsweise steht
- X: für den neutralen Säurerest einer n acide Wasserstoffatome aufweisenden, Sauerstoff enthaltenden Säure eines maximalen pKa-Wertes von 2.

Geeignet sind beispielsweise entsprechende silylierte Sulfonsäuren wie Trifluormethansulfonsäuretrimethylsilylester oder Methansulfonsäuretrimethylsilylester, silylierte Ester von Säuren des Phosphors wie Phosphorsäuretris(trimethylsilylester) oder Phosphorsäurediethylester-trimethylsilylester.

Die Menge des erfindungswesentlichen Katalysatorgifts liegt im allgemeinen bei 0,01 bis 2, vorzugsweise 0,5 bis 1,5 Mol pro Mol an eingesetztem Katalysator. Die optimale Menge des Katalysatorgifts kann durch einen orientierenden Vorversuch ermittelt werden. Hinzugabe des Katalysatorgifts erfolgt innerhalb des Temperaturbereichs von 0 bis 100, vorzugsweise 20 bis 80°C.

Nach Abbruch der Reaktion durch Zusatz des erfindungswesentlichen Katalysatorgifts kann gewünschtenfalls nicht umgesetztes monomeres Ausgangspolyisocyanat durch beliebige Trennoperationen wie z.B. Destillation, insbesondere Dünnschichtdestillation oder Extratkion, bei Feststoffen durch Filtration, entfernt werden. Es kann zur erneuten Modifizierung ohne Beeinträchtigung der Aktivität wieder eingesetzt werden. Oftmals erfolgt die Entfernung des nichtumgesetzten Ausgangspolyisocyanats bis auf einen Restgehalt von maximal 1 Gew.-%.

Neben der Wiederverwendbarkeit des Ausgangsisocyanats weisen die erfindungsgemäßen Produkte den Vorteil auf, eine hohe Lagerstabilität zu besitzen und verbesserte Farbwerte bis hin zu farblosen Produkten aufzuweisen.

Die erfindungsgemäßen Verfahrensprodukte stellen wertvolle Ausgangsmaterialien zur Herstellung von Polyurethankunststoffen, insbesondere zur Herstellung von Polyurethanlacken und Polyurethanklebstoffen dar. Sie können hierbei sowohl in von überschüssigem Ausgangspolyisocyanat weitgehend befreiter Form als auch in Form ihrer Lösungen in überschüssigem Ausgangspolyisocyanat als auch, gewünschtenfalls, in mit an sich bekannten Blockierungsmitteln blockierter Form in an sich bekannter Weise zum Einsatz gelangen.

Die in den nachfolgenden Beispielen genannten Teile beziehen sich auf Gewichtsteile und die Prozentangaben auf Gewichtsprozente.

### Beispiele

### Beispiel 1

Beispiel 1 zeigt die erfindungswesentliche, verbesserte Abstopperwirkung von Trifluormethansulfonsäuretrimethylsilylester im Vergleich zu Toluol-4-sulfonyl-isocyanat (Tosylisocyanat).

840 g (5 Mol) 1,6-Diisocyanatohexan (HDI) werden unter Rühren und Stickstoffatmosphäre auf 60°C erwärmt und mit 2 g 2-Ethylhexanol-1 und 2 g Tri-n-butylphosphin (10 mMol) versetzt. Das Reaktionsgemisch wird anschließend bei 60°C gerührt, wobei gleichzeitig der Reaktionsfortgang über die zunehmenden Brechungsindices verfolgt wird. Nach 4 Stunden bei 60°C ist ein Brechungsindex (23°C) von 1,4610 erreicht (Ausgangswert: 1,4520).

Die Hälft der Rohlösung wird sofort mit 11 g (5 mMol) einer 10 %igen Lösung von Trifluormethansulfonsäuretrimethylsilylester in HDI abgestoppt (Lösung A), die andere Hälfte mit 9,9 g (5 mMol) einer 10 %igen Lösung von Tosylisocyanat in HDI versetzt (Lösung B).
Lösung A war selbst nach 10 Tagen Lagerung bei 60°C stabil (n_{D} (23°C):1,4610) und farblos. Lösung B war bereits nach 7 Stunden Lagerung bei 60°C von n_{D} (23°C) 1,4610 auf 1,4650 angestiegen und tiefgelb gefärbt.

### Beispiel 2

840 g (5 Mol) HDI werden gemäß Beispiel 1 mit 2 g 2-Ethylhexanol-1 und 2,5 g Tri-n-butylphosphin (12,4 mMol) zur Reaktion gebracht. Nach 6 1/2 Stunden bei 60°C ist ein Brechungsindex (23°C) von 1,4663 erreicht. Man stoppt mit 0,9 g (4 mMol) Trifluormethansulfonsäuretrimethylsilylester ab und befreit das Produkt durch Dünnschichtdestillation bei 160°C/0,1 mbar bis auf einen Restgehalt von 0,2 % von überschüssigem HDI. Es resultiert ein klares, fast farbloses Uretdion- und Isocyanuratgruppen aufweisendes Polyisocyanat mit einem NCO-Gehalt von 22,1 % und einer Viskosität (23°C) von 210 mPas.s. Die Ausbeute, bezogen auf ursprünglich eingesetztes HDI beträgt 43 %. Gehalt an freiem HDI: 0,2 %.

### Beispiel 3

Eine Lösung von 150 g (0,86 Mol) 2,4-Diisocyanatotoluol in 850 g Chlorbenzol wird mit 0,2 g (1 mMol) Tri-n-butylphosphin bei 25°C zur Reaktion gebracht. Nach 30 Minuten bei 25°C beginnt sich ein farbloser Niederschlag auszubilden der kontinuierlich wächst. Nach 7 Stunden bei 25°C wird mit 0,1 g Trifluormethansulfonsäuretrimethylsilylester (0,45 mMol) abgestoppt. Der Niederschlag setzt sich über Nacht ab, die überstehende Lösung hat ihren Brechungsindex über Nacht beibehalten (n_{D} 823°C): 1,5266). Man saugt ab und erhält nach Trocknung ein farbloses, kristallines Produktmit den Daten:
Ausbeute: 75 g (50 %)
NCO-Gehalt: 23,4 %

## Patentansprüche

1. Verfahren zur Herstellung von oligomeren Polyisocyanaten durch Oligomerisierung eines Teils der Isocyanatgruppen von organischen Polyisocyanaten in Gegenwart von die Dimerisierung von Isocyanatgruppen beschleunigenden Katalysatoren und Abbruch der Oligomerisierungsreaktion bei einem Umsatz von 5 bis 45 % der im Ausgangsgemisch vorliegenden Isocyanatgruppen durch Zugabe eines Katalysatorengifts, dadurch gekennzeichnet, daß man als Katalysatorengift eine silysierte Säure der Formel
X-[Si(CH₃)₃]ₙ
verwendet, wobei
X für den neutralen Säurerest steht, wie er durch Entfernung der aciden Wasserstoffatome aus einer n-basischen Säure mit einem pKa-Wert von maximal 3 erhalten wird, wobei Halogenwasserstoffsäuren ausgenommen sind, und
n für eine ganze Zahl von 1 bis 3 steht.

2. Verfahren gemäß Anspruch 1, dadurch gekenzeichnet, daß man als Katalysator tertiäre Phosphine des Molekulargewichtsbereichs 76 bis 500 verwendet.

3. Verfahren gemäß Anspruch 1 und 2, dadurch gekennzeichnet, daß man als Katalysatorgift der Formel gemäß Anspruch 1 entsprechende, O-silylierte, Sauerstoff enthaltende Säuren verwendet, die in nicht-silylierter Form einen pKa-Wert von maximal 2 aufweisen.

4. Verfahren gemäß Anspruch 1 bis 3, dadurch gekennzeichnet, daß man als Katalysatorgift Trifluormethansulfonsäuretrimethylsilylester verwendet.

5. Verfahren gemäß Anspruch 1 bis 4, dadurch gekennzeichnet, daß man als Ausgangspolyisocyanat 1,6-Diisocyanatohexan, 1-Isocyanato-3,3,5-trimethyl-5-isocyanatomethyl-cyclohexan, 4,4'-Diisocyanatodicyclohexylmethan oder beliebige Gemische dieser Diiocyanate verwendet.

6. Verfahren gemäß Anspruch 5, dadurch gekennzeichnet, daß man die oligomeren Polyisocyanate nach Abbruch der Dimerisierungsreaktion durch Dünnschichtdestillation von überschüssigen Mengen des Ausgangspolyisocyanats bis auf einen Restgehalt von maximal 1 Gew.-% befreit.

7. Verfahren gemäß Anspruch 1 bis 4, dadurch gekennzeichnet, daß man als organisches Ausgangspolyisocyanat aromatische Diisocyanate, ausgewählt aus der Gruppe bestehend aus (i) 2,4- und/oder 2,6-Diisocyanatotoluol, (ii) 2,2'-, 2,4- und/oder 4,4'-Diisocyanatodiphenylmethan und (iii) beliebigen Gemischen dieser Diisocyanate verwendet.

8. Verwendung der gemäß Anspruch 1 bis 7 erhaltenen, oligomeren Polyisocyanate, gegebenenfalls im Gemisch mit nicht umgesetztem Ausgangspolyisocyanat und/oder gegebenenfalls in mit Blockierungsmitteln für Isocyanatgruppen blockierter Form als Polyisocyanatkomponente bei der Herstellung von Polyurethankunststoffen nach dem Isocyanat-Polyadditionsverfahren.

## Claims

1. Process for the production of oligomeric polyisocyanates by oligomerising a proportion of the isocyanate groups of organic polyisocyanates in the presence of catalysts which accelerate the dimerisation of isocyanate groups and terminating the oligomerisation reaction at a conversion of 5 to 45% of the isocyanate groups present in the initial mixture by adding a catalyst poison, characterised in that the catalyst poison used is a silylated acid of the formula
X-[Si(CH₃)₃]ₙ
wherein
X denotes the neutral acid residue as obtained by removing the acidic hydrogen atom from an n-basic acid having a pKa value of at most 3, wherein hydrohalic acids are excepted, and
n denotes an integer from 1 to 3.

2. Process according to claim 1, characterised in that tertiary phosphines of the molecular weight range from 76 to 500 are used as the catalyst.

3. Process according to claims 1 and 2, characterised in that O-silylated acids containing oxygen and of the formula according to claim 1, which have a pKa value of at most 2 in non-silylated from, are used as the catalyst poison.

4. Process according to claims 1 to 3, characterised in that trifluoromethanesulphonic acid trimethylsilyl ester is used as the catalyst poison.

5. Process according to claims 1 to 4, characterised in that 1,6-diisocyanatohexane, 1-isocyanato-3,3,5-trimethyl-5-isocyanatomethylcyclohexane, 4,4'-diisocyanatodicyclohexylmethane or any desired mixtures of these diisocyanates are used as the starting polyisocyanate.

6. Process according to claim 5, characterised in that, once the dimerisation reaction has been terminated, excess quantities of the starting polyisocyanates are removed from the oligomeric polyisocyanates by film distillation down to a residual content of at most 1 wt.%.

7. Process according to claims 1 to 4, characterised in that aromatic diisocyanates selected from the group comprising (i) 2,4- and/or 2,6-diisocyanatotoluene, (ii) 2,2'-, 2,4- and/or 4,4'-diisocyanatodiphenylmethane and (iii) any desired mixtures of these diisocyanates are used as the organic starting polyisocyanate.

8. Use of the oligomeric polyisocyanates obtained according to claims 1 to 7, optionally mixed with unreacted starting polyisocyanate and/or optionally blocked with blocking agents for isocyanate groups as a polyisocyanate component in the production of polyurethane plastics using the isocyanate polyaddition process.

## Revendications

1. Procédé de préparation de polyisocyanates oligomères par oligomérisation d'une partie des groupes isocyanates de polyisocyanates organiques en présence de catalyseurs accélérant la dimérisation des groupes isocyanates et interruption de la réaction d'oligomérisation à un taux de conversion de 5 à 45% des groupes isocyanates présents dans le mélange initial par addition d'un poison de catalyseur, caractérisé en ce que l'on utilise en tant que poison de catalyseur un acide silylé de formule
X-[Si(CH₃)₃]ₙ
dans laquelle
X représente un radical d'acide neutre tel qu'obtenu par élimination des atomes d'hydrogène acides dans un acide n-basique présentant une valeur de pKa de 3 au maximum, les hydracides halogénés étant exclus, et
n est un nombre entier allant de 1 à 3.

2. Procédé selon la revendication 1, caractérisé en ce que l'on utilise en tant que catalyseurs des phosphines tertiaires de poids moléculaire 76 à 500.

3. Procédé selon les revendications 1 et 2, caractérisé en ce que l'on utilise en tant que poisons du catalyseur à la formule de la revendication 1, des acides oxygénés O-silylés qui, à l'état non silylé, ont une valeur de pKa de 2 au maximum.

4. Procédé selon les revendications 1 à 3, caractérisé en ce que l'on utilise en tant que poison de catalyseur le trifluorométhanesulfonate de triméthylsilyle.

5. Procédé selon les revendications 1 à 4, caractérisé en ce que l'on utilise en tant que polyisocyanate de départ le 1,6-diisocyanatohexane, le 1-isocyanato-3,3,5-triméthyl-5-isocyanatométhyl-cyclohexane, le 4,4'-diisocyanatodicyclohexylméthane ou un mélange quelconque de ces diisocyanates.

6. Procédé selon la revendication 5, caractérisé en ce que, après avoir arrêté la réaction de dimérisation, on débarrasse les polyisocyanates oligomères de l'excès du polyiisocyanate de départ par distillation en couche mince jusqu'à une teneur résiduelle de 1% en poids au maximum.

7. Procédé selon les revendications 1 à 4, caractérisé en ce que l'on utilise en tant que polyisocyanates organiques de départ des diisocyanates aromatiques choisis dans le groupe consistant en (i) le 2,4- et/ou le 2,6-diisocyanatotoluène, (ii) le 2,2'-, le 2,4'- et/ou le 4,4'diisocyanatodiphénylméthane et (iii) des mélanges quelconques de ces diisocyanates.

8. Utilisation des polyisocyanates oligomères obtenus selon les revendications 1 à 7, le cas échéant en mélange avec le polyisocyanate de départ non converti et/ou le cas échéant à l'état bloqué par des agents bloquants des groupes isocyanates, en tant que composants polyisocyanates à la préparation de résines synthétiques de polyuréthane par le procédé de polyaddition des isocyanates.
